# EUROPEAN PATENT APPLICATION

(11) **EP 4 623 909 A1**
(43) Date of publication of application: **01.10.2025**
(21) Application number: 23905781.3
(22) Date of filing: 13.12.2023
(51) Int. Cl.: A61K 31/4015, A61K 31/53, C07D 413/12, C07D 487/04, A61P 31/14

(54) **PHARMACEUTICAL COMPOSITION FOR TREATING OR RELIEVING COVID-19 AND FORMULATION COMPRISING SAME**

(30) Priority: 23.12.2022 CN 202211662655
(71) Applicant: Guangdong Raynovent Biotech Co., Ltd., Huangpu District Guangzhou 510700 (CN); Shenzhen Antiv Pharma Co., Ltd., Shenzhen, Guangdong 518118 (CN)
(72) Inventor: LI, Guanguan, Shenzhen, Guangdong 518118 (CN); CHEN, Xiaoxin, Guangzhou, Guangdong 510700 (CN); ZHOU, Qifan, Shenzhen, Guangdong 518118 (CN); CAO, Liu, Shenzhen, Guangdong 518118 (CN); HUANG, Jianzhou, Guangzhou, Guangdong 510700 (CN); LONG, Chaofeng, Guangzhou, Guangdong 510700 (CN); YANG, Sidi, Shenzhen, Guangdong 518118 (CN); PENG, Chao, Shenzhen, Guangdong 518118 (CN); LI, Kun, Shenzhen, Guangdong 518118 (CN)
(74) Representative: Clark, Claudia
(86) International application number: PCT/CN2023/138496
(87) International publication number: WO 2024/131615

(57) **Abstract**

The present invention relates to a pharmaceutical composition comprising a first active ingredient and a second active ingredient, wherein the first active ingredient is compound 1, an ester corresponding thereto, a salt corresponding thereto, a salt of the ester corresponding thereto, or a combination of the substances, and the second active ingredient is ATV014, or a pharmaceutically acceptable salt, a hydrate, or a solvate thereof. The molar ratio of the first active ingredient to the second active ingredient is 50: 1-1:50. The composition has relatively good prospects for pharmaceutical development.

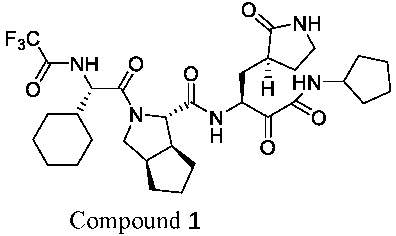

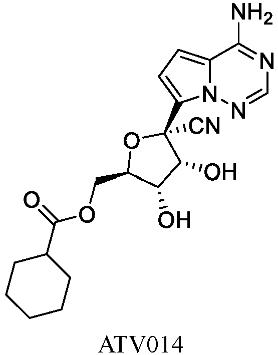

## Description

### TECHNICAL FIELD

The present disclosure belongs to the field of pharmaceutical formulations, and particularly relates to a pharmaceutical composition for treating or relieving novel coronavirus pneumonia and a formulation comprising the pharmaceutical composition.

### BACKGROUND

Novel coronavirus pneumonia (COVID-19) has become one of the most serious infectious diseases in human history, which is caused by Severe Acute Respiratory Syndrome Coronavirus 2 (SARS-CoV-2), and has infected more than 650 million people. It is the public health problem which is most urgently needs to be solved at present in the world.

Although vaccination and antiviral drugs are currently available for treatment, the emergence of variant strains has caused recurrent epidemics. Therefore, the development of new safe and effective antiviral drugs is of great significance for curbing the epidemics. Patent PCT/CN2022/117124 discloses a series of keto-amide derivatives with 3CL protease binding inhibition. The in vitro activity data shows that some of the compounds exhibit positive effects in the inhibitory activity assay targeting the novel coronavirus main protease (Mpro, 3CL). Some of the compounds also exhibit longer half-life and better pharmacokinetic properties in the further pharmacokinetic assays in mice. The comprehensive performance of Compound 1 (Example 1 of PCT/CN2022/117124) is relatively outstanding, and is considered to possess a high prospect for drug development.

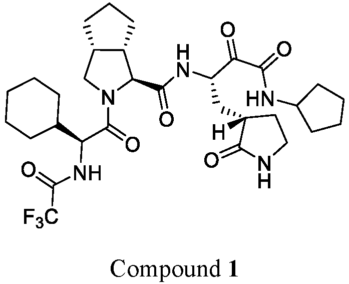

ATV014, CAS: 2691076-98-7, developed by the team of Prof. Xumu Zhang at Southern University of Science and Technology, is a nucleoside compound that can inhibit RNA-dependent RNA polymerase (RdRp). It has good in vitro antiviral activity, pharmacokinetic properties, in vivo antiviral effect, and a broad-spectrum anti-coronavirus activity.

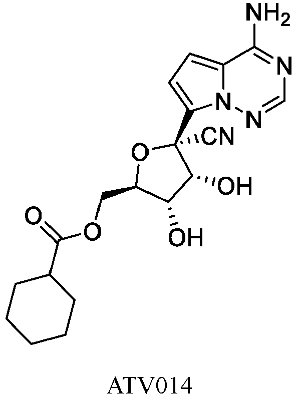

The genetic material RNA genome of the novel coronavirus may automatically mutate at any time during its proliferation, and the enzyme that repairs the error mechanism in the genetic material RNA of the novel coronavirus during replication has low activity and cannot correct the error in time, thus leading to the possibility of mutation of novel coronavirus in a short time. In addition, because the external environment is constantly changing, in order to adapt to the new environment, novel coronavirus constantly enhances its adaptability to the environment, increasing the possibility of gene mutation of the novel coronavirus replicon, and leading to the mutation of the novel coronavirus within a short time. At the same time, due to the clinical use of antiviral drugs for symptomatic treatment, in order to survive, the novel coronavirus may also have a gene mutation in a short time, or become more toxic and enhance drug resistance.

It can be seen that the novel coronavirus has the characteristics of a high mutation rate and many recombination phenomena between viruses, which affect the therapeutic effect of drugs. Although active compounds with new structures and mechanisms are constantly being discovered, in the long run, the use of a single antiviral drug may not be able to effectively inhibit/reduce the concentration of the virus in the body within a short time and cure novel coronavirus pneumonia. Furthermore, the corresponding increase in the dose of the drug and the elongation of the treatment duration also bring a greater medication risk to patients. Combined administration is considered as one of the effective solutions.

Paxlovid is the world's first oral 3CL protease inhibitor for novel coronavirus launched by Pfizer, which comprises two components, one of which is PF-07321332 (nirmatrelvir), and the other is ritonavir. PF-07321332 inhibits the activity of 3CL (the main protease required for the replication of novel coronavirus). Ritonavir slows down the decomposition of PF-07321332 (the first component in the body), allowing PF-07321332 to increase in concentration and maintain a long-lasting effect, thus improving its effectiveness.

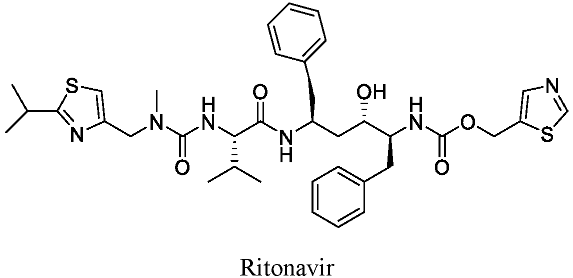

It is an urgent technical problem in the existing technology to find a novel solution for enhancing the activity of 3CL protease inhibitors, overcoming the drug resistance of viruses and prolonging the service life of anti-novel coronavirus pneumonia drugs with promising clinical applications.

### SUMMARY

The first objective of the present disclosure is to overcome the deficiencies in the existing technology, and to provide a pharmaceutical composition for treating novel coronavirus pneumonia. The pharmaceutical composition solves the technical problems, such as drug resistance of novel coronavirus and reduces the risk of clinical medication by utilizing the synergistic effect of active ingredients.

The objective of the present disclosure is achieved by the following technical solutions.

The first aspect of the present disclosure provides a pharmaceutical composition comprising a first active ingredient and a second active ingredient, wherein the first active ingredient is Compound **1,** an ester of Compound **1,** a salt of Compound **1,** a salt of the ester of Compound **1,** or a combination thereof; and the second active ingredient is ATV014, or a pharmaceutically acceptable salt, a hydrate, or a solvate thereof. Specifically, a molar ratio of the first active ingredient to the second active ingredient ranges from 50:1 to 1:50.

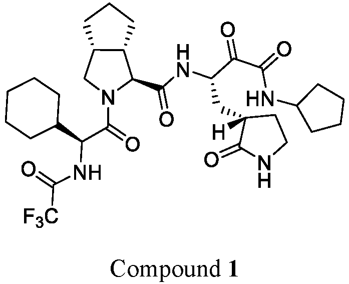

The first active ingredient refers to Compound **1,** an ester of Compound **1,** a salt of Compound **1,** a salt of the ester of Compound **1,** or a combination thereof, and specifically may be any one of Compound **1,** an ester of Compound **1,** a salt of Compound **1,** a salt of the ester of Compound **1,** or a combination of any two or more in any proportion thereof. Each of the aforementioned Compound **1,** the ester of Compound **1,** the salt of Compound **1,** or the salt of the ester of Compound **1** respectively comprises its specific terms such as anhydrate, non-solvate, hydrate and solvate. The ester of Compound **1** refers to an ester formed by Compound **1** with an organic acid, including but not limited to formate, acetate, propionate, isopropionate, n-butyrate tert-butyrate of Compound **1,** and the like. The salt of Compound **1** refers to a salt formed by Compound **1** with an organic acid and/or an inorganic acid, or a salt formed by Compound **1** with an organic base and/or an inorganic base, including but not limited to a hydrochloride, a hydrobromide, a sulfate, a phosphate, a benzenesulfonate, a p-toluenesulfonate, a methanesulfonate, a tartrate, a camphorsulfonate, a lithium salt, a sodium salt, a potassium salt, a calcium salt, a magnesium salt, an aluminum salt, an ammonia salt, an ethylenediamine salt, an triethylamine salt of Compound **1,** and the like. The salt of the ester of Compound **1** refers to a salt formed by the ester of Compound **1** with an organic base and/or an inorganic base and/or an organic acid and/or an inorganic acid, including but not limited to a hydrochloride of methyl ester of Compound **1,** a sulfate of methyl ester of Compound **1,** a hydrochloride of ethyl ester of Compound **1,** a sulfate of ethyl ester of Compound **1,** and the like.

The second active ingredient is ATV014 or a pharmaceutically acceptable salt, a hydrate, or a solvate thereof.

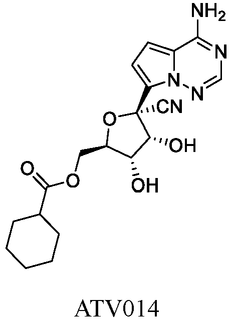

The second active ingredient refers to ATV014 or a pharmaceutically acceptable salt thereof, and also comprises its specific terms such as anhydrate, non-solvate, hydrate and solvate. For example, ATV014 may be an anhydrate of ATV014, a hydrate of ATV014, a solvate of ATV014, and the like.

The molar ratio of the first active ingredient to the second active ingredient ranges from 50:1 to 1:50. Preferably, the molar ratio of the first active ingredient to the second active ingredient ranges from 20:1 to 1:20. More preferably, the molar ratio of the first active ingredient to the second active ingredient ranges from 10:1 to 1:10. More preferably, the molar ratio of the first active ingredient to the second active ingredient ranges from 5:1 to 1:10. Most preferably, the molar ratio of the first active ingredient to the second active ingredient is 1:1.

In some specific embodiments, the molar ratio of the first active ingredient to the second active ingredient is 50:1, 40:1, 30:1, 20:1, 10:1, 5:1, 3:1, 2:1, 1.5:1, 1:1, 1:1.5, 1:2, 1:3, 1:5, 1:10, 1:20, 1:30, 1:40, or 1:50.

Unless otherwise indicated, the mass of the salt of the present disclosure refers to the mass calculated in free species (free base/acid equivalent), and the mass of the hydrate/solvate refers to the mass based on dry basis and pure basis (on an anhydrous basis).

The inventors surprisingly found in experiments that the pharmaceutical composition comprising the first active ingredient (Compound **1,** or an ester of Compound **1,** or a salt of Compound **1,** or a salt of the ester of Compound **1,** or a combination thereof) and the second active ingredient (ATV014, or a pharmaceutically acceptable salt, a hydrate, or a solvate thereof) has a good pharmaceutical synergy effect when the molar ratio of the two active ingredients is within a specific range, which is manifested as a significantly better anti-novel coronavirus effect than a single ingredient, and also manifested as a significantly better anti-novel coronavirus effect than a pharmaceutical composition with a molar ratio falling outside of the specific range described above. It can be seen that the pharmaceutical composition has a preferable anti-novel coronavirus effect when the molar ratio of the first and the second active ingredients in a unit formulation is within the specific range. Based on the evaluation of other properties (such as stability and fluidity of the mixture), it is concluded that the pharmaceutical composition has higher prospect of being developed into a medicine.

In addition, the embodiment can also be used for combined administration, such as simultaneous or sequential administration of the first active ingredient and the second active ingredient. When the molar ratio of the two active ingredients is within a specific range, said pharmaceutical composition has a good pharmaceutical synergy effect, which is manifested as a significantly better anti-novel coronavirus effect than a single ingredient, and also manifested as a significantly better anti-novel coronavirus effect than a pharmaceutical composition with a molar ratio falling outside of the specific range described above.

Further, the pharmaceutical composition comprising a first active ingredient and a second active ingredient, according to the aforementioned first objective of the present disclosure, further comprises a third active ingredient, wherein the third active ingredient is an antiretroviral drug. The definitions and the corresponding ranges of the first active ingredient and the second active ingredient are the same as those of the pharmaceutical composition according to the aforementioned first objective of the present disclosure. Specifically, the third active ingredient refers to the antiretroviral drug ritonavir.

Specifically, in the pharmaceutical composition comprising the third active ingredient, as previously described, the first active ingredient is Compound **1,** or an ester of Compound **1,** or a salt of Compound **1,** or a salt of the ester of Compound **1,** or a combination thereof; and the second active ingredient is ATV014, or a pharmaceutically acceptable salt thereof; and a molar ratio of the first active ingredient to the second active ingredient to the third active ingredient in the pharmaceutical composition ranges from approximately 50:1:0.1-5 to 1:50:0.1-5. Preferably, the molar ratio of the first active ingredient to the second active ingredient to the third active ingredient in the pharmaceutical composition ranges from 20:1:0.3-2 to 1:20:0.3-2. Preferably, the molar ratio of the first active ingredient to the second active ingredient to the third active ingredient in the pharmaceutical composition ranges from 10:1:0.3-2 to 1:10:0.3-2. More preferably, the molar ratio of the first active ingredient to the second active ingredient to the third active ingredient in the pharmaceutical composition ranges from 5:1:0.3-2 to 1:10:0.3-2. More preferably, the molar ratio of the first active ingredient to the second active ingredient to the third active ingredient in the pharmaceutical composition ranges from 1:1:0.3-2.

Specifically, the molar ratio of the first active ingredient to the second active ingredient to the third active ingredient in the pharmaceutical composition is approximately 50:1:1, 40:1:1, 30:1:1, 24:1:1, 20:1:1, 10:1:1, 6:1:1, 5:1:1, 3:1:1, 2:1:1, 1.5:1:1, 1:1:1, 1:1.5:1, 1:2:1, 1:3:1, 1:5:1, 1:6:1, 1:10:1, 1:20:1, 1:24:1, 1:30:1, 1:40:1, or 1:50:1.

In a preferred embodiment of the present disclosure, the aforementioned pharmaceutical composition comprising a first active ingredient, a second active ingredient and a third active ingredient is a combination of Compound **1,** ATV014, and ritonavir.

In some preferred embodiments, the first active ingredient and second active ingredient are present in separate units of formulation, respectively.

In some preferred embodiments, the first active ingredient and second active ingredient are administered simultaneously or sequentially.

Another objective of the present disclosure is to provide a preparation method of the aforementioned pharmaceutical composition. The preparation method can ensure stable preparation of the aforementioned pharmaceutical composition. Specifically, the preparation method is prepared by a conventional mixing method in the art. More specifically, the preparation method may be prepared by a direct mixing method, an equivalent incremental method, and the like. The equipment used in the mixing process may be common mixing equipment in the art, such as V-type mixer, double cone mixer, rotary mixer, etc., depending on the scale of the preparation. Manual mixing may also be used for small-scale preparation.

The third objective of the present disclosure is to provide a formulation comprising the aforementioned pharmaceutical composition. Specifically, the formulation comprises the aforementioned pharmaceutical composition and a pharmaceutically acceptable excipient, wherein the pharmaceutically acceptable excipient is any one or more selected from the group consisting of a filler, a binder, a disintegrant, a glidant, a lubricant, a flavoring agent, etc, or in any combination of two or more thereof.

Specifically, the filler is any one or more selected from the group consisting of calcium carbonate, magnesium carbonate, calcium phosphate, calcium sulfate, magnesium oxide, calcium carboxymethylcellulose, sodium carboxymethylcellulose, sucrose, lactose, fructose, xylitol, mannitol, starch or derivatives thereof, dextrin, microcrystalline cellulose, etc., or in any combination of two or more in any portion thereof.

Specifically, the binder is any one or more selected from the group consisting of gum Arabic, gelatin, tragacanth, dextrin, polyvinylpyrrolidone, starch and derivatives thereof, sodium alginate, sorbitol, syrup, hydroxypropyl methylcellulose, methylcellulose, hydroxypropyl cellulose, hydroxyethyl cellulose, ethyecellulose, sodium carboxymethylcellulose, calcium carboxymethylcellulose, glucose, polymethacrylate, etc., or in any combination of two or more in any portion thereof.

Specifically, the disintegrant is any one or more selected from the group consisting of starch, alginate, calcium carboxymethylcellulose, sodium carboxymethylcellulose, croscarmellose sodium, crospolyvinylpyrrolidone, low-substituted hydroxypropyl methylcellulose, microcrystalline cellulose, methylcellulose, etc., or in any combination of two or more in any portion thereof.

Specifically, the glidant is any one or more selected from the group consisting of colloidal silicon dioxide, powdered cellulose, magnesium trisilicate, silicon dioxide, talcum powder, etc., or in any combination of two or more in any portion thereof.

Specifically, the lubricant is any one or more selected from the group consisting of calcium stearate, glycerol monostearate, glycerol palmitostearate, magnesium stearate, microcrystalline cellulose, sodium benzoate, sodium chloride, sodium dodecyl sulfate, magnesium stearate, sodium stearyl fumarate, talcum powder, zinc stearate, polyethylene glycol, etc., or in any combination of two or more in any portion thereof.

Specifically, the flavoring agent is any one or more selected from the group consisting of stevioside, aspartame, other flavors and sweeteners commonly used in the art, etc., or in any combination of two or more in any portion thereof.

In some preferred embodiments, the formulation comprising the aforementioned pharmaceutical composition is an oral formulation, which may be in the form of powder, granule, pellet, capsule, tablet, lozenge or oral liquid.

In the aforementioned formulation, the specification of Compound **1,** or the ester of Compound **1,** or the salt of Compound **1,** or the salt of the ester of Compound **1,** or the combination thereof in the formulation, i.e., the amount of Compound **1,** or the ester of Compound **1,** or the salt of Compound **1,** or the salt of the ester of Compound **1,** or the combination thereof contained per unit of the formulation, may be from 50 mg to 1,500 mg. Preferably, the specification of the formulation ranges from 100 mg to 1,000 mg. Specifically, the specification of Compound **1,** or the ester of Compound **1,** or the salt of Compound **1,** or the salt of the ester of Compound **1,** or the combination thereof in the formulation is 100 mg, 150 mg, 200 mg, 300 mg, 400 mg, 500 mg, 600 mg, 700 mg, 800 mg, 900 mg or 1,000 mg.

In the composition of the present disclosure, the first active ingredient and the second active ingredient may be present in separate units of formulation, respectively. The first active ingredient and the second active ingredient may be administered simultaneously or sequentially.

Based on the beneficial effects of the aforementioned pharmaceutical composition, such as pharmaceutical synergy effect, etc., the formulation containing the aforementioned pharmaceutical composition also correspondingly exhibits significantly better anti-novel coronavirus effect than a formulation containing a single ingredient, and also significantly better anti-novel coronavirus effect than a formulation containing a pharmaceutical composition with a molar ratio falling outside of the specific range described above. The formulation containing the aforementioned pharmaceutical composition has a good market prospect.

The fourth objective of the present disclosure is to provide a pharmaceutical use, that is, use of the pharmaceutical composition according to the aforementioned first objective of the present disclosure and/or the formulation according to aforementioned third objective of the present disclosure in the manufacture of a medicament for relieving or treating a novel coronavirus infection.

The fifth objective of the present disclosure is to provide a use. The first active ingredient and the second active ingredient are formulated to be separately administered at a frequency of once a day, twice a day or three times a day, for daily administration. The first active ingredient and second active ingredient are formulated to be co-delivered in a common orally administered dose unit, or the first active ingredient and the second active ingredient are formulated to be administered in separate orally administered dose units.

The inventors surprisingly found that, based on the synergistic effect between drugs, the pharmaceutical composition described herein and the formulation comprising the pharmaceutical composition exhibit better anti-novel coronavirus therapeutic effects than individual ingredient, which can effectively reduce the dose and contribute to the avoidance of the emergence of drug resistance. Further, the novel coronavirus infection aimed by the pharmaceutical use is a novel coronavirus infection caused by a common type of novel coronavirus and a variant thereof. More further, the novel coronavirus infection aimed by the pharmaceutical use is a novel coronavirus infection caused by an Alpha variant strain, a Beta variant strain, a Gamma variant strain, a Delta variant strain, a Lambda variant strain and/or an Omicron variant strain. The infection comprises fever, cough, sore throat, pneumonia, acute respiratory infection, severe acute respiratory infection, hypoxic respiratory failure, acute respiratory distress syndrome, sepsis or septic shock.

In some preferred embodiments, the first active ingredient and the second active ingredient are separately formulated, and separately administered at a frequency of once a day, twice a day or three times a day.

In some preferred embodiments, the first active ingredient and second active ingredient are formulated to be co-delivered in orally administered dose units.

In some preferred embodiments, the first active ingredient and the second active ingredient are formulated to be administered in separate orally administered dose units.

The present disclosure has the following outstanding advantages and beneficial effects over the existing technology:
1. The present provides a pharmaceutical composition comprising a first active ingredient (Compound **1,** an ester of Compound **1,** a salt of Compound **1,** a salt of the ester of Compound **1,** or a combination thereof) and a second active ingredient (ATV014 or a pharmaceutically acceptable salt thereof) with a molar ratio in a specific range, and optionally further comprising a third active ingredient (an antiretroviral drug). The pharmaceutical composition has a good pharmaceutical synergistic effect, and is considered to possess a high prospect for drug development after comprehensive evaluation.
2. The present provides a preparation method of the pharmaceutical composition, which can ensure stable preparation of the pharmaceutical composition described above.
3. The present disclosure provides a formulation comprising the aforementioned pharmaceutical composition, which also correspondingly exhibits significantly better anti-novel coronavirus effect than a formulation containing a single ingredient, and also significantly better anti-novel coronavirus effect than a formulation containing a pharmaceutical composition with a molar ratio falling outside of the specific range described above, and has a good market prospect.
4. The present disclosure provides a pharmaceutical which utilizes synergistic effects between drugs to achieve better therapeutic effects.

The contribution of the present disclosure lies in the discovery of a synergistic effect between drugs in a specific range, and therefore it can be understood by those skilled in the art that when the types and proportions of the active ingredients in the dosing unit of unit are within the range described in the present disclosure, or when the types and proportions of the dosing unit of unit of different active ingredients in the same unit of sale are within the range described in the present disclosure, it can be regarded as the use of the technical embodiment protected by the present disclosure. Specifically, the aforementioned dosing unit of unit refers to the smallest unit for clinical use, such as a tablet per unit of tablet, a capsule per unit of capsule, an oral liquid per unit of bottle, a granule per unit of packet, etc. The aforementioned dosing unit of unit of different active ingredients in the same unit of sale means that different dosing unit of units are placed in the same package for sale according to a specific ratio, and different dosing unit of units are taken and administrated in combination according to a specific ratio in clinical use. For example, tablets (such as Paxlovid) with different active ingredients are placed in a unit box, etc.

### Definitions and general terms

Some embodiments of the present disclosure are now described in detail, examples of which are illustrated by the accompanying structural and chemical formulae. The present disclosure is intended to cover all alternatives, modified and equivalent technical solutions which are included within the scope of the present disclosure as defined by the claims. Those skilled in the art would recognize that many methods and materials similar or equivalent to those described herein can be used in practicing the present disclosure. The present disclosure is not limited to the methods and materials described herein. In the event that one or more of the combined literature, patents, and similar materials are different or contradictory to this application (including, but not limited to, the terms defined, the application of terms, the techniques described, and the like), this application shall prevail.

It should be further recognized that certain features of the present disclosure, which are described in several independent embodiments for the sake of clarity, but may also be provided in combination in a single example. Otherwise, various features of the present disclosure are described in a single embodiment for the sake of brevity, but may also be provided individually or in any suitable subcombination.

The term "comprises/comprising" is an open-ended expression, i.e., it includes what is indicated in the present disclosure, but does not exclude other aspects.

"Pharmaceutically acceptable salt", as used herein, refers to organic salts and inorganic salts of compounds in the present disclosure. Pharmaceutically acceptable salts are well known in the art to which they belong, for example, those described in S. M. Berge et al., describe pharmaceutically acceptable salts in detail in J. Pharmaceutical Sciences, 1977, 66: 1-19. Pharmaceutically acceptable non-toxic salts formed with acids include, but are not limited to, inorganic acid salts such as hydrochloride, hydrobromide, phosphate, sulfate, and perchlorate; and organic acid salts such as acetate, oxalate, maleate, tartrate, citrate, succinate, and malonate; or salts obtained by using other methods described in the literature such as ion exchange. Other pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, and valerate, and the like. Salts obtained by reaction with appropriate bases include alkali metal, alkaline earth metal, ammonium and N⁺ (C₁₋₄ alkyl)₄ salts. The present disclosure further contemplates quaternary ammonium salts formed from any compounds comprising N groups. Water-soluble or oil-soluble or dispersible products may be obtained by quaternization. Alkali or alkaline earth metal salts include sodium, lithium, potassium, calcium, and magnesium salts, and the like. Pharmaceutically acceptable salts further include appropriate and non-toxic ammonium, quaternary ammonium salts, and amine cations formed with counterions, such as halide, hydroxide, carboxylate, sulfate, phosphate, nitrate, C₁₋₈ sulfonate, and aromatic sulfonate.

"Solvate" of the present disclosure refers to an association substance formed by one or more solvent molecules with a compound of the present disclosure. Solvents that form solvates include, but are not limited to, isopropanol, ethanol, methanol, dimethyl sulfoxide, ethyl acetate, acetic acid and aminoethanol.

The term "hydrate" refers to an association substance formed by one or more water molecules with a compound of the present disclosure.

"Combined/combination" denotes a fixed combination in the form of a single dose unit or a kit of portions for combined administration, wherein the disclosed compound of the present disclosure and the combination partner may be administered independently at the same time or may be administered separately within a certain time interval, in particular to make the combination and the partner exhibit cooperative, e.g., synergistic effects. As used herein, the terms "pharmaceutical composition" or "combined administration", etc., are intended to encompass the administration of a selected combination partner to a single individual (e.g., a patient) in need thereof, and are intended to encompass therapeutic regimens in which the substances do not have to be administered by the same route of administration or simultaneously.

### BRIEF DESCRIPTION OF THE DRAWINGS

FIG. 1 shows curves of weight change in infected mice after administration; and
FIG. 2 shows a graph of viral load in the lungs after administration for different time.

### DETAILED DESCRIPTION

The present disclosure is described in further detail below with examples, but the embodiments of the present disclosure are not limited to these.

The ATV014 described in the present disclosure may be prepared with reference to the methods disclosed in the existing technology, such as the methods in Example 9 and Example 17 of CN 202111083730.9 and Example 1 to Example 3 of CN 202110621245.6, respectively.

Specifically, ATV014 used in the examples listed in the present disclosure is a self-made active pharmaceutical ingredient (API) with a purity of >99% according to the method described above.

Ritonavir used in the examples is an active pharmaceutical ingredient (API) for the commercially available formulation ritonavir tablets, CAS: 155213-67-5, with a purity of >99%.

### Example 1: Preparation of Compound 1

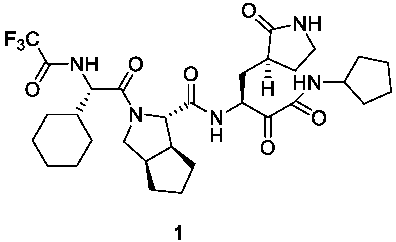

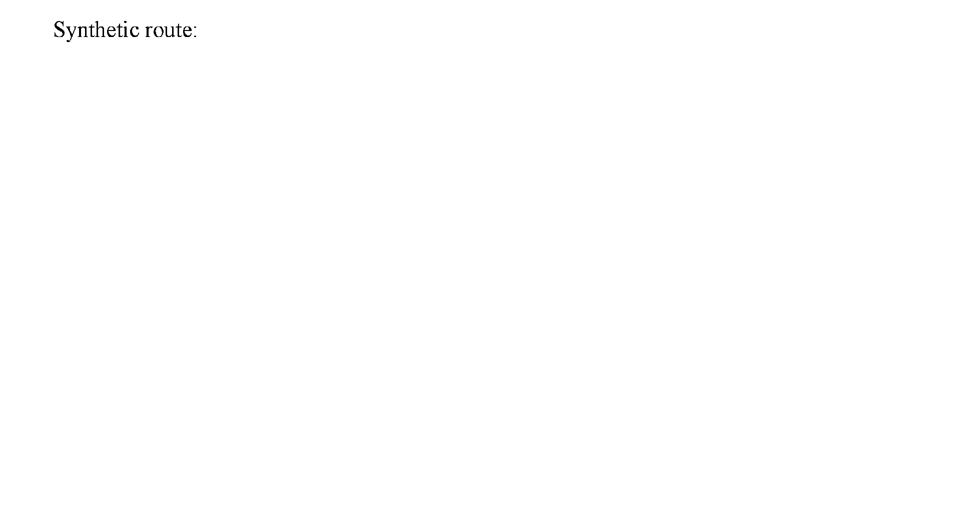

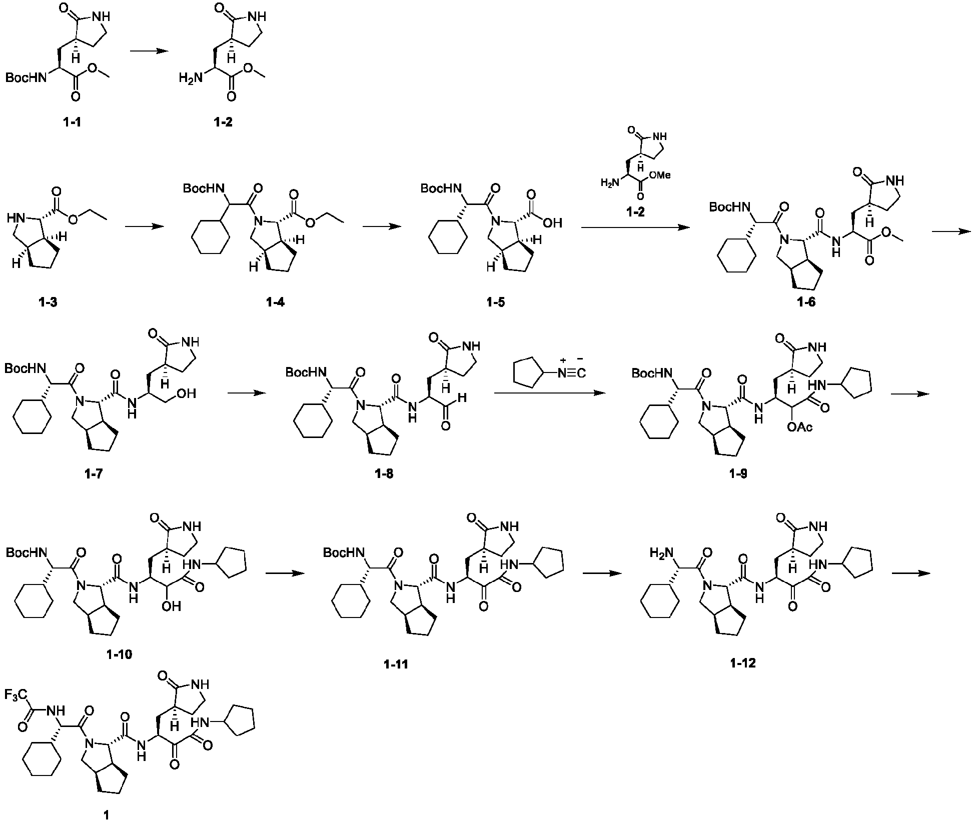

### Step 1: Synthesis of hydrochloride of Compound 1-2

Compound **1-1** (500 mg, 1.75 mmol) was dissolved in ethyl acetate (5 mL), followed by the addition of a solution of hydrogen chloride in ethyl acetate (10 mL, 4 N). The reaction mixture was stirred at 20 °C for 2 hours (h), concentrated under reduced pressure to obtain the hydrochloride of Compound **1-2** without further purification. ¹H NMR (400 MHz, CD₃OD) δ = 4.28 - 4.20 (m, 1 H), 3.91 - 3.81 (m, 3 H), 3.45 - 3.35 (m, 2 H), 2.86 - 2.74 (m, 1 H), 2.48 - 2.36 (m, 1 H), 2.29 - 2.19 (m, 1 H), 2.02 - 1.94 (m, 1 H), 1.93 - 1.80 (m, 1 H).

### Step 2: Synthesis of Compound 1-4

Compound Boc-L-cyclohexylglycine (1 g, 3.89 mmol) was added to N,N-dimethylformamide (10 mL), followed by the addition of 2-(7-azabenzotriazol-1-yl)-*N,N,N',N'*-tetramethyluronium hexafluorophosphate (1.77 g, 4.66 mmol). The reaction mixture was stirred for 0.5 h. Diisopropylethylamine (1.26 g, 9.72 mmol) and hydrochloride of Compound **1-3** (1.02 g, 4.66 mmol) were added, and the reaction mixture was stirred at 20 °C for 16 h. To the reaction solution was added methyl tert-butyl ether (50 mL), washed with water (20 mL), washed with 3% citric acid (20 mL × 2), and washed with saturated sodium chloride solution (20 mL). The organic layer was dried over anhydrous sodium sulfate, filtered and concentrated under reduced pressure, and then purified by silica gel column chromatography (petroleum ether: ethyl acetate = 3:1) to obtain Compound **1-4.** ¹H NMR (400 MHz, CDCl₃) δ = 5.22 - 5.11 (m, 1 H), 4.36 (d, J=3.9 Hz, 1 H), 4.27 (dd, J=6.9, 9.3 Hz, 1 H), 4.21 - 4.12 (m, 2 H), 3.83 (dd, J=7.8, 10.4 Hz, 1 H), 3.70 (br dd, J=3.6, 10.4 Hz, 1 H), 2.81 - 2.61 (m, 2 H), 1.82 - 1.70 (m, 6 H), 1.68 - 1.61 (m, 4 H), 1.56 - 1.48 (m, 2 H), 1.46 - 1.38 (m, 9 H), 1.29 - 1.22 (m, 4 H), 1.21 - 0.98 (m, 4 H).

### Step 3: Synthesis of Compound 1-5

Compound **1-4** (1.41 g, 3.34 mmol) was added to tetrahydrofuran (14 mL), followed by the addition of a solution of lithium hydroxide monohydrate, LiOH·H₂O (280.03 mg, 6.67 mmol) in water (5 mL). The reaction mixture was stirred at 20 °C for 16 h. The crude product was neutralized with 3 % citric acid solution (50 mL), extracted with ethyl acetate (50 mL), and the organic phase was washed with saturated sodium chloride solution (30 mL). The organic layer was dried over anhydrous sodium sulfate, filtered and concentrated under reduced pressure to obtain Compound **1-5** without further purification. ¹H NMR (400 MHz, DMSO-d₆) δ = 12.58 - 12.23 (m, 1 H), 6.92 - 6.82 (m, 1 H), 4.11 - 3.94 (m, 2 H), 3.82 - 3.76 (m, 1 H), 3.72 - 3.62 (m, 1 H), 2.73 - 2.64 (m, 1 H), 2.62 - 2.55 (m, 1 H), 1.92 - 1.42 (m, 12 H), 1.40 - 1.32 (m, 9 H), 1.18 - 1.06 (m, 3 H), 1.00 - 0.81 (m, 2 H).

### Step 4: Synthesis of Compound 1-6

Compound **1-5** (650 mg, 1.65 mmol) was added to 2-butanone (7 mL), followed by the addition of 1-hydroxybenzotriazole (222.63 mg, 1.65 mmol), 1-(3-dimethylaminopropyl)-3-ethylcarbodiimide hydrochloride (379.03 mg, 1.98 mmol), and diisopropylethylamine (638.84 mg. 4.94 mmol). The reaction mixture was stirred at 20 °C for 0.5 h. The hydrochloride of Compound **1-2** (366.88 mg, 1.65 mmol) was added, and the reaction mixture was stirred at 20 °C for 16 h. To the reaction solution was added water (20 mL), and extracted with the mixture of dichloromethane and methanol (30 mL×2, 10:1), and the organic phases were combined. The organic phase was washed with 3 % citric acid (20 mL×2), and washed with saturated sodium chloride solution (20 mL). The organic layer was dried over anhydrous sodium sulfate, filtered and concentrated under reduced pressure, and then purified by silica gel column chromatography (dichloromethane: methanol= 20:1) to obtain Compound **1-6.** ¹H NMR (400 MHz, CDCl₃) δ = 7.49 - 7.42 (m, 1 H), 6.23 - 6.05 (m, 1 H), 5.28 - 5.17 (m, 1 H), 4.64 - 4.51 (m, 1 H), 4.43 - 4.24 (m, 2 H), 3.92 - 3.81 (m, 1 H), 3.78 - 3.70 (m, 3 H), 3.39 - 3.27 (m, 2 H), 2.94 - 2.75 (m, 2 H), 2.57 - 2.36 (m, 2 H), 2.24 - 2.07 (m, 1 H), 1.94 - 1.50 (m, 14 H), 1.49 - 1.41 (m, 9 H), 1.27 - 0.95 (m, 6 H).

### Step 5: Synthesis of Compound 1-7

Compound **1-6** (3.10 g, 5.51 mmol) was dissolved in tetrahydrofuran (31 mL), followed by the addition of lithium borohydride (240.02 mg, 11.02 mmol) at 0 °C, and the temperature was slowly raised to 20 °C for 2 h. To the reaction solution were added water (10 mL) and ethyl acetate (20 mL), and was stirred for 10 min. A white solid was precipitated and filtered to obtain a filter cake, i.e., the crude product of the target product Compound **1-7.** [M + 1] ⁺ = 535.4.

### Step 6: Synthesis of Compound 1-8

Compound 1-7 (0.5 g, 935.13 µmol) was dissolved in dichloromethane (10 mL), then Dess-Martin oxidant (594.94 mg, 1.40 mmol) was added to the reaction system. The reaction mixture was stirred at 25 °C for 16 h. To the reaction system were added saturated sodium thiosulfate solution (15 mL) and saturated sodium bicarbonate solution (15 mL), and stirred for 10 min, extracted with dichloromethane (50 mL×2). The organic phase was washed with saturated saline solution (15 mL), dried over anhydrous sodium sulfate, filtered, and concentrated to obtain the crude product of Compound **1-8.** [M + 1] ⁺ = 533.4.

### Step 7: Synthesis of Compound 1-9

Compound **1-8** (436 mg, 818.52 µmol) was dissolved in dichloromethane (5 mL), and glacial acetic acid (58.98 mg, 982.22 mmol), cyclopentyl isocyanide (94.44 mg, 982.22 µmol) was added to the reaction system. The reaction mixture was stirred at 25 °C for 2 h. To the reaction system was added saturated ammonium chloride solution (10 mL), and stirred for 10 min, extracted with dichloromethane (20 mL). The organic phase was washed with water (10 mL), dried over anhydrous sodium sulfate, filtered and concentrated under reduced pressure, and then purified by silica gel column chromatography (dichloromethane: methanol = 10:1) to obtain Compound **1-9.** [M + 1] ⁺ = 688.4.

### Step 8: Synthesis of Compound 1-10

Compound **1-9** (190 mg, 276.22 µmol) was dissolved in methanol (3 mL), and then a solution of potassium carbonate (95.44 mg, 690.54 µmol) in water (2 mL) was added. The reaction mixture was stirred at 20 °C for 16 h. To the reaction system was added 3% citric acid (20 mL), extracted with dichloromethane (40 mL) for 3 times. The organic phase was washed with saturated sodium chloride solution (30 mL), dried over anhydrous sodium sulfate, filtrated, and concentrated under reduced pressure to obtain the crude product of Compound **1-10.** [M + 1] ⁺ = 646.5.

### Step 9: Synthesis of Compound 1-11

Compound **1-10** (238.00 mg, 368.52 µmol) was dissolved in dichloromethane (24 mL), and then Dess-Martin oxidant (203.19 mg, 479.08 µmol) was added. The reaction mixture was stirred at 20 °C for 18 h. To the reaction system were added sodium thiosulfate (15 mL) and sodium bicarbonate solution (15 mL), and stirred for 10 min, extracted with dichloromethane (50 mL×2). The organic phase was washed with saturated saline solution (15 mL), dried over anhydrous sodium sulfate, filtered and concentrated, and then purified by silica gel column chromatography (dichloromethane: methanol= 20:1) to obtain the product Compound **1-11.** [M + 1] ⁺ = 644.5.

### Step 10: Synthesis of Compound 1-12

Compound **1-11** (125 mg, 194.16 µmol) was dissolved in tetrahydrofuran (3 mL) and then ethyl acetate hydrochloride (4 M, 2.91 mL) was added. The reaction mixture was stirred at 20 °C for 1 h. The reaction solution was evaporated directly with an oil pump and was repeatedly evaporated with a small amount of dichloromethane to obtain Compound **1-12.** [M + 1] ⁺ =544.4.

### Step 11: Synthesis of Compound 1

Compound **1-12** (125 mg, 229.91 µmol) were dissolved in tetrahydrofuran (2.5 mL), followed by the addition of trifluoroacetic anhydride (193.15 mg, 919.63 µmol), and pyridine (127.30 mg, 1.61 mmol) at 0 °C. The reaction mixture was stirred at 20 °C for 16 h. To the reaction system was added water (20 mL), extracted with dichloromethane (40 mL×2). The organic phase was washed with 3 % citric acid (40 mL) and saturated sodium chloride solution (40 mL×2) sequentially, dried over anhydrous sodium sulfate, filtered and concentrated. The crude product was isolated by preparative HPLC to obtain Compound **1.** [M + 1] ⁺ = 640.0, ¹H NMR (400 MHz, CD₃OD) δ ppm 0.94 - 1.10 (m, 2 H), 1.13 - 1.32 (m, 3 H) 1.32 - 1.46 (m, 1 H), 1.47 - 1.57 (m, 3 H), 1.59 - 1.68 (m, 4 H), 1.69 - 1.81 (m, 6 H), 1.83 - 2.00 (m, 5 H), 2.01 - 2.17 (m, 1 H), 2.19 - 2.38 (m, 1 H), 2.49 - 2.57 (m, 1 H), 2.58 - 2.70 (m, 1 H), 2.73 - 2.89 (m, 1 H), 3.20 - 3.26 (m, 1 H), 3.37 - 3.45 (m, 1 H), 3.73 - 3.86 (m, 1 H), 3.88 - 3.97 (m, 1 H), 4.03 - 4.10 (m, 1 H), 4.11 - 4.18 (m, 1 H), 4.19 - 4.29 (m, 1 H), 4.29 - 4.37 (m, 1 H), 4.39 - 4.47 (m, 1 H), 4.57 - 4.60 (m, 2 H).

### Example 2

1.00 g of the free form of Compound **1** prepared in Example 1 was taken and mixed homogeneously with 0.94 g of ATV014 to obtain a pharmaceutical composition with a molar ratio of 1:1.5.

### Example 3.

1.00 g of the free form of Compound **1** prepared in Example 1 was taken and mixed homogeneously with 1.25 g of ATV014 to obtain a pharmaceutical composition with a molar ratio of 1:2.

### Example 4

1.00 g of the free form of Compound **1** prepared in Example 1 was taken and mixed homogeneously with 1.88 g of ATV014 to obtain a pharmaceutical composition with a molar ratio of 1:3.

### Example 5

0.50 g of the free form of Compound **1** prepared in Example 1 was taken and mixed homogeneously with 1.57 g of ATV014 to obtain a pharmaceutical composition with a molar ratio of 1:5.

### Example 6

0.50 g of the free form of Compound **1** prepared in Example 1 was taken and mixed homogeneously with 3.14 g of ATV014 to obtain a pharmaceutical composition with a molar ratio of 1:10.

### Example 7

0.10 g of the free form of Compound **1** prepared in Example 1 was taken and mixed homogeneously with 3.14 g of ATV014 to obtain a pharmaceutical composition with a molar ratio of 1:50.

### Example 8

1.00 g of the free form of Compound **1** prepared in Example 1 was taken and mixed homogeneously with 0.63 g of ATV014 to obtain a pharmaceutical composition with a molar ratio of 1:1.

### Example 9.

1.00 g of the free form of Compound **1** prepared in Example 1 was taken and mixed homogeneously with 0.42 g of ATV014 to obtain a pharmaceutical composition with a molar ratio of 1.5:1.

### Example 10

2.00 g of the free form of Compound **1** prepared in Example 1 was taken and mixed homogeneously with 0.63 g of ATV014 to obtain a pharmaceutical composition with a molar ratio of 2:1.

### Example 11

2.00 g of the free form of Compound **1** prepared in Example 1 was taken and mixed homogeneously with 0.42 g of ATV014 to obtain a pharmaceutical composition with a molar ratio of 3:1.

### Example 12

2.00 g of the free form of Compound **1** prepared in Example 1 was taken and mixed homogeneously with 0.25 g of ATV014 to obtain a pharmaceutical composition with a molar ratio of 5:1.

### Example 13

2.00 g of the free form of Compound **1** prepared in Example 1 was taken and mixed homogeneously with 0.13 g of ATV014 to obtain a pharmaceutical composition with a molar ratio of 10:1.

### Example 14

5.00 g of the free form of Compound **1** prepared in Example 1 was taken and mixed homogeneously with 0.06 g of ATV014 to obtain a pharmaceutical composition with a molar ratio of 50:1.

### Example 15

The pharmaceutical compositions with molar ratios of the free form of Compound 1 to ATV014 of 40:1, 30:1, 20:1, 1:20, 1:30 and 1:40 were prepared following the same method as described in Example 2, respectively.

### Example 16

2.00 g of the free form of Compound **1** prepared in Example 1 was taken and mixed homogeneously with 0.42 g of ATV014 and 0.75 g of ritonavir to obtain a pharmaceutical composition with a molar ratio of 3:1:1.

### Example 17: Test of the anti-novel coronavirus activity of the composition of Compound 1 and ATV014

Experimental objective: To test the anti-novel coronavirus activity of the composition of Compound 1 and ATV014 by cellular assay.

Virus strain for the experiment: SARS-CoV-2 (B.1, hCoV-19/CHN/SYSU-IHV/2020 strain, Accession ID on GITHE: EPI_ISL_444969).

Experimental conditions: all experiments were done in the Biosafety Level-3 Laboratory of Sun Yat-sen University.

### Experimental scheme:

Two 24-well plates of A549-hACE2 cells were seeded with 5×10⁴ cells per well. After 24 h of cell adherence, the cells were infected by SARS-CoV-2 at 0.05 of multiplicity of infection (MOI). After 1 h of infection at 37°C, the cells were washed twice with pre-warmed PBS solution. The compositions of Compound **1** and ATV014 prepared from Examples 2 to 16 were configured into different concentrations, and then the infected cells were added to the medium containing different concentrations of the drugs to be tested or DMSO. After 48 h, the supernatant was collected, and the intracellular viruses were detected by fluorescence quantitative PCR, and the inhibition rate of the drugs was calculated (see Table 1).

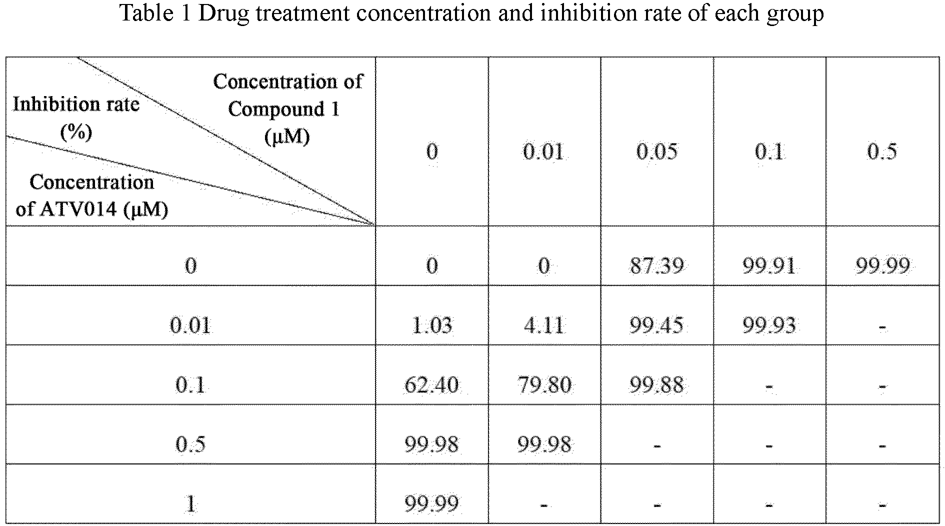

Note: "-" indicates that the experimental data has failed to reflect the effect of the combined administration effect and thus the details are not repeated herein.

### Results:

The inhibition rate reached 99.91% when Compound **1** was used alone at a concentration of 0.1 µM. As the concentration of Compound 1 increased, there was no significant increase in inhibition rate. The inhibition rate reached 99.98% when ATV014 was used alone at a concentration of 0.5 µM. As the concentration of ATV014 increased, there was no significant increase in inhibition rate.

No antiviral effect was observed when Compound **1** was used alone at a concentration of 0.01 µM. The inhibition rate was 62.40% when ATV014 was used alone at a concentration of 0.1 µM. However, when 0.01 µM Compound **1** was combined with 0.1 µM ATV014 for administration, the virus inhibition rate increased to 79.80%. The inhibition effect showed a significant increase with the increasing concentration of ATV014, which demonstrated a significant synergistic effect.

The inhibition rate reached 87.39% when Compound **1** was used alone at a concentration of 0.05 µM. There was almost no antiviral effect when ATV014 was used alone at a concentration of 0.01 µM. However, when 0.05 µM Compound **1** was combined with 0.01 µM ATV014 for administration, the viral inhibition rate increased to 99.45%, which demonstrated a significant synergistic effect.

The other groups also showed significant synergistic effects.

In summary, the combined administration of Compound **1** and ATV014 enhances the antiviral effect at the cellular level. In vitro activity experiments shows that Compound **1** and ATV014 have a certain synergistic effect in vitro, and have a better anti-novel coronavirus effect than the experimental groups using Compound **1** alone and ATV014 alone.

Further in vivo activity experiments show that Compound **1** or an ester of Compound **1** or a salt of Compound **1** or a salt of the ester of Compound **1** or a combination thereof, and ATV014 or a pharmaceutically acceptable salt thereof have a certain synergistic effect, which is manifested that the combined administration of the two have better anti-novel coronavirus effect than that used alone.

To sum up, it can be seen from the results of the activity experiment that when the molar ratio of Compound **1** or an ester of Compound **1** or a salt of Compound **1 or** a salt of the ester of Compound **1** or a combination thereof, and ATV014 or a pharmaceutically acceptable salt thereof ranges from 1:50 to 50:1, there is a good pharmaceutical synergy effect between the active ingredients, which is manifested as a significantly better anti-novel coronavirus effect than a single ingredient, and also manifested as a significantly better anti-novel coronavirus effect than a pharmaceutical composition with a molar ratio falling outside of the specific range described above. Specifically, when the molar ratio of Compound **1** or an ester of Compound **1** or a salt of Compound **1** or a salt of the ester of Compound **1** or a combination thereof, and ATV014 or a pharmaceutically acceptable salt thereof is 10:1, 5:1, 3:1, 2:1, 1.5:1, 1:1, 1:1.5, 1:2, 1:3, 1:5, or 1:10, the anti-novel coronavirus effect of the composition is significantly superior to that of Compound **1** (or an ester of Compound **1,** or a salt of Compound **1,** or a salt of the ester of Compound **1,** or a combination thereof) used alone, and is significantly superior to that of ATV014 (or a pharmaceutically acceptable salt thereof) used alone, and is also significantly superior to that of pharmaceutical compositions whose molar ratios are not within this specific range.

The activity experiments further show that the pharmaceutical composition has a good anti-novel coronavirus effect when the molar ratio of Compound **1** or an ester of Compound **1** or a salt of Compound **1** or a salt of the ester of Compound **1** or a combination thereof, and ATV014 or a pharmaceutically acceptable salt thereof, and ritonavir ranges from 50:1:0.1-5 to 1:50:0.1-5. The pharmaceutical composition has a better anti-novel coronavirus effect when the molar ratio of the first active ingredient, the second active ingredient and the third active ingredient in the pharmaceutical composition ranges from approximately 20:1:0.3-2 to 1:20:0.3-2.The pharmaceutical composition has an optimal anti-novel coronavirus effect when the molar ratio of the first active ingredient, the second active ingredient and the third active ingredient in the pharmaceutical composition ranges from approximately 10:1:0.3-2 to 1:10:0.3-2.

### Example 18

The pharmaceutical composition obtained in Example 2 was mixed with an appropriate amount of filler, binder and disintegrant, and then prepared by direct compression process into a tablet (the mass combination of Compound **1** and ATV014 is: 1.00 mg + 0.94 mg).

The pharmaceutical composition obtained in Example 3 was mixed with an appropriate amount of filler, binder, disintegrant, and then prepared by direct compression process into a tablet (the mass combination of Compound **1** and ATV014 is: 1.00 mg + 1.25 mg).

The pharmaceutical composition obtained in Example 4 was mixed with an appropriate amount of filler, binder, disintegrant, and then prepared by direct compression process into a tablet (the mass combination of Compound **1** and ATV014 is: 1.00 mg + 1.88 mg).

The pharmaceutical composition obtained in Example 5 was mixed with an appropriate amount of filler, binder, disintegrant, and then prepared by direct compression process into a tablet (the mass combination of Compound **1** and ATV014 is: 0.50 mg + 1.57 mg).

The pharmaceutical composition obtained in Example 6 was mixed with an appropriate amount of filler, binder, disintegrant, and then prepared by direct compression process into a tablet (the mass combination of Compound **1** and ATV014 is: 0.50 mg + 3.14 mg).

The pharmaceutical composition obtained in Example 7 was mixed with an appropriate amount of filler, binder, disintegrant, and then prepared by direct compression process into a tablet (the mass combination of Compound **1** and ATV014 is: 0.10 mg + 3.14 mg).

The pharmaceutical composition obtained in Example 8 was mixed with an appropriate amount of filler, binder, disintegrant, and then prepared by direct compression process into a tablet (the mass combination of Compound **1** and ATV014 is: 1.00 mg + 0.63 mg).

The pharmaceutical composition obtained in Example 9 was mixed with an appropriate amount of filler, binder, disintegrant, and then prepared by direct compression process into a tablet (the mass combination of Compound **1** and ATV014 is: 1.00 mg + 0.42 mg).

The pharmaceutical composition obtained in Example 10 was mixed with an appropriate amount of filler, binder, disintegrant, and then prepared by direct compression process into a tablet (the mass combination of Compound **1** and ATV014 is: 2.00 mg + 0.63 mg).

The pharmaceutical composition obtained in Example 11 was mixed with appropriate amount of filler, binder, disintegrant, and then prepared by direct compression process into a tablet (the mass combination of Compound **1** and ATV014 is: 2.00 mg + 0.42 mg).

The pharmaceutical composition obtained in Example 12 was mixed with an appropriate amount of filler, binder, disintegrant, and then prepared by direct compression process into a tablet (the mass combination of Compound **1** and ATV014 is: 2.00 mg + 0.25 mg).

The pharmaceutical composition obtained in Example 13 was mixed with an appropriate amount of filler, binder, disintegrant, and then prepared by direct compression process into a tablet (the mass combination of Compound **1** and ATV014 is: 2.00 mg + 0.13 mg).

The pharmaceutical composition obtained in Example 14 was mixed with an appropriate amount of filler, binder, disintegrant, and then prepared by direct compression process into a tablet (the mass combination of Compound **1** and ATV014 is: 5.00 mg + 0.06 mg).

The pharmaceutical composition obtained in Example 16 was mixed with an appropriate amount of filler, binder, disintegrant, and then prepared by direct compression process into a tablet (the mass combination of Compound **1,** ATV014, and ritonavir is: 2.00 mg + 0.42 mg + 0.75 mg).

### Example 19

200 mg of Compound **1** was mixed with an appropriate amount of filler, binder, and disintegrant, and then prepared by direct compression process into a tablet. 188 mg of ATV014 was mixed with an appropriate amount of filler, binder, and disintegrant, and then prepared by direct compression process into a tablet. The two types of tablets are placed in the same package.

200 mg of Compound **1** was mixed with an appropriate amount of filler, binder, disintegrant, and then prepared by direct compression process into a tablet. 250 mg of ATV014 was mixed with an appropriate amount of filler, binder, disintegrant, and then prepared by direct compression process into a tablet. The two types of tablets are placed in the same package.

200 mg of Compound **1** was mixed with an appropriate amount of filler, binder, disintegrant, and then prepared by direct compression process into a tablet. 376 mg of ATV014 was mixed with an appropriate amount of filler, binder, disintegrant, and then prepared by direct compression process into a tablet. The two types of tablets are placed in the same package.

200 mg of Compound **1** was mixed with an appropriate amount of filler, binder, disintegrant, and then prepared by direct compression process into a tablet. 628 mg of ATV014 was mixed with an appropriate amount of filler, binder, disintegrant, and then prepared by direct compression process into a tablet. The two types of tablets are placed in the same package.

200 mg of Compound **1** was mixed with appropriate amount of filler, binder, disintegrant, and then prepared by direct compression process into a tablet. 126 mg of ATV014 was mixed with appropriate amount of filler, binder, disintegrant, and then prepared by direct compression process into a tablet. The two types of tablets are placed in the same package.

200 mg of Compound **1** was mixed with an appropriate amount of filler, binder, disintegrant, and then prepared by direct compression process into a tablet. 84 mg of ATV014 was mixed with an appropriate amount of filler, binder, disintegrant, and then prepared by direct compression process into a tablet. The two types of tablets are placed in the same package.

200 mg of Compound **1** was mixed with appropriate amount of filler, binder and disintegrant, and then prepared by direct compression process into a tablet. 63 mg of ATV014 was mixed with appropriate amount of filler, binder and disintegrant, and then prepared by direct compression process into a tablet. The two types of tablets are placed in the same package.

200 mg of Compound **1** was mixed with appropriate amount of filler, binder and disintegrant, and then prepared by direct compression process into a tablet. 42 mg of ATV014 was mixed with appropriate amount of filler, binder and disintegrant, and then prepared by direct compression process into a tablet. The two types of tablets are placed in the same package.

200 mg of Compound **1** was mixed with appropriate amount of filler, binder, disintegrant, and then prepared by direct compression process into a tablet. 25 mg of ATV014 was mixed with appropriate amount of filler, binder, disintegrant, and then prepared by direct compression process into a tablet. The two types of tablets are placed in the same package.

200 mg of Compound **1** was mixed with an appropriate amount of filler, binder, disintegrant, and then prepared by direct compression process into a tablet. 13 mg of ATV014 was mixed with an appropriate amount of filler, binder, disintegrant, and then prepared by direct compression process into a tablet. The two types of tablets are placed in the same package.

266 mg of Compound **1** was mixed with an appropriate amount of filler, binder, disintegrant, and then prepared by direct compression process into a tablet. 56 mg of ATV014 was mixed with an appropriate amount of filler, binder, disintegrant, and then prepared by direct compression process into a tablet. The two types of tablets are placed in the same package with a tablet of 100 mg of ritonavir.

### Example 20

This test was performed according to the same process as in Example 17. The experimental data showed that the combination of Compound **1,** ATV014 and ritonavir had a high viral inhibition rate, indicating that Compound **1,** ATV014 and ritonavir had a significant drug synergistic effect. The pharmaceutical composition exhibits a good inhibitory effect on novel coronavirus when the molar ratio of Compound **1** to the second active ingredient of ATV014 to the third active ingredient of ritonavir ranges from approximately 50:1:0.1-5 to 1:50:0.1-5. The pharmaceutical composition exhibits a good inhibitory effect on novel coronavirus when the molar ratio of the first active ingredient to the second active ingredient to the third active ingredient ranges from approximately 20:1:0.3-2 to 1:20:0.3-2. The pharmaceutical composition exhibits a good inhibitory effect on novel coronavirus when the molar ratio of the first active ingredient to the second active ingredient to the third active ingredient ranges from approximately 20:1:0.3-2 to 1:20:0.3-2.The pharmaceutical composition exhibits a more better inhibitory effect on novel coronavirus when the molar ratio of the first active ingredient to the second active ingredient to the third active ingredient ranges from approximately 10:1:0.3-2 to 1:10:0.3-2. It is preferred that the pharmaceutical composition with a molar ratio of 1:1:0.3-2 exhibits the optimal inhibitory effect on novel coronavirus when the molar ratio of the first active ingredient to the second active ingredient to the third active ingredient ranges from approximately 5:1:0.3-2 to 1:10:0.3-2.

Specifically, the above molar ratio of the first active ingredient to the second active ingredient to the third active ingredient may be 50:1:1, 40:1:1, 30:1:1, 24:1:1, 20:1:1, 10:1:1, 6:1:1, 5:1:1, 3:1:1, 2:1:1, 1.5:1:1, 1:1:1, 1:1:1,1:1:1, 1:1.5:1,1:2:1,1:3. 1, 1:5:1, 1:6:1, 1:10:1, 1:20:1, 1:24:1, 1:30:1, 1:40:1, or 1:50:1.

### Example 21: Test of the in vivo anti-novel coronavirus activity of the composition of Compound 1 and ATV014

Experimental objective: To test the in vivo anti-novel coronavirus activity of the composition of Compound **1** and ATV014 by modeling novel coronavirus infection in mice.

### Experimental scheme:

### 1. Materials and methods

### 1.1 Main instruments

The main instruments used in this experiment were listed below:

**Table 2 Main instruments**

| Instrument name | Brand | Model |
|---|---|---|
| IVC mouse cage | Tecniplast | ISO72NFFNZ |
| Secondary biological safety cabinet | Tecniplast | TECH60 |
| Tissue grinder | TIANGEN | TGinder H24 tissue |
| Carbon dioxide incubator | THERMO ELECTRON | FORMA 3111 |
| 37 °C incubator | BOXUN | BSC-400 |
| Immuno ELISPOT detector | CTL-Immuno Spot | S6 Ultra |
| Real-time fluorescence quantitative PCR instrument | ABI | Applied Biosystems QuantStudio 7 |

### 1.2 Software

GraphPad Prism 8.0.1 software was used in this experiment for result data plotting, summarizing and statistics.

### 1.3 Main experimental reagents

**Table 3 Information of the compounds to be tested**

| Name of the compound to be tested | Structural formula | Source |
|---|---|---|
| ATV014 | 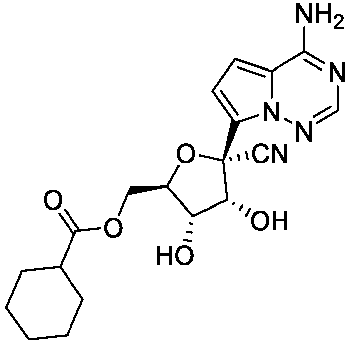 | Shenzhen Antiv Pharma Co., Ltd. |
| Compound 1 | 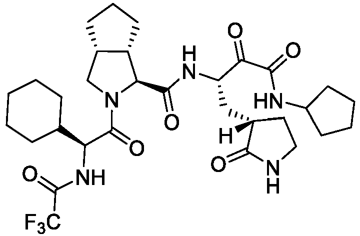 | Guangdong Raynovent Biotech Bo., Ltd. |

**Table 4 Information of main reagents**

| Reagent name | Manufacturer | Batch No. |
|---|---|---|
| Triton-X 100 | Sigma | T8787-100mL |
| SARS-CoV N rabbit polyclonal antibody | Sino Biological | 40143-T62 |
| HRP goat-anti rabbit IgG | abcam | ab6721 |
| DMEM medium | CORNING | 10-013-CV |
| 4% paraformaldehyde | biosharp | BL539A |
| 1.6% CMC | Sigma | C4888-500G |
| True-blue chromogenic solution | KPL | 50-78-02 |
| DPBS | CORNING | 21-031-CVO |
| Isoflurane | Beifute | 20210901 |
| Mineral oil | Solarbio | M8040 |
| qPCR kit | Daan Gene | DA0930 |

### 1.4 Experimental strains, cells, and mice

Delta strain of novel coronavirus was isolated and preserved by Guangdong CDC Biosafety Level-3 Laboratory, K18-hACE2 transgenic mice were provided by the School of Medicine of Sun Yat-sen University and purchased from Jackson Laboratory (USA), and the breed was C57BL/6 (B6.Cg-Tg(K18-ACE2)2Prlmn/J).

### 2 Test contents and methods

### 2.1 Animal grouping

The mice (C57BL/6(B6.Cg-Tg(K18-ACE2)2Prlmn/J)) to be tested were 40 in total, and were randomly grouped according to their weights before the experiment, with 4 groups of 10 mice in each group. The group settings were as follows:

**Table 5 Animal group settings**

| Group | Number of animals/femal e (mouse) | Subject or control | Dose (PFU /mouse) | Dose/time (mg/kg) | Daily dose (mg/kg) | Subject concentration (mg/mL) | Administration dose (mL/mouse) |
|---|---|---|---|---|---|---|---|
| 1 | 10 | Vehicle | 500 | 0 | 0 | 0 | 0.2 |
| 2 | 10 | ATV014 | 500 | 100 | 100 | 10 | 0.2 |
| 3 | 10 | Compound 1 | 500 | 150 | 150 | 15 | 0.2 |
| 4 | 10 | Compound 1 +ATV014 | 500 | 150/100 | 150/100 | 15/10 | 0.2 |

### 2.2 Modeling of novel coronavirus infection in mice.

Forty C57BL/6 (B6.Cg-Tg(K18-ACE2)2Prlmn/J) mice were anesthetized with isoflurane and infected with the novel coronavirus Delta strain by nasal drip. The dose of the strain nasal drops was 500 PFU/mouth, and the infection volume was 50 µL.

### 2.3 Drug treatment of mice

### Blank vehicle group:

Prescription composition: 20% [40% SBE-β-CD] + 20 % HS15 + 60% water, pH = 4

The mice were infected by nose drops at day 0, and the vehicle given at a volume of 0.2 mL/mouse was initiated 2 hours post-challenge, once daily until post-challenge day 3 and day 5.

5 mL of Compound **1** solution was prepared with a concentration of 30 mg/mL of Compound 1.

15 mL of ATV014 solution was prepared with a concentration of 10 mg/mL of ATV014.

**Table 6 Material List for Preparation Dose Groups of Test Sample**

| Material | Mass or volume |
|---|---|
| Compound **1** (30 mg/mL) | 150 mg |
| ATV014 (10 mg/mL) | 150 mg |
| 40% sulfobutyl-β-cyclodextrin (40% SBE-β-CD) | 5 mL |
| polyethylene glycol (15)-hydroxystearate (HS15) | 5 mL |
| 1 M hydrochloric acid | 1 mL |
| water | 13 mL |
| 1 M sodium hydroxide solution | about 0.9 mL |

### Preparation steps:

1. Placing Compound **1** (150 mg) into a mortar, adding HS15 (5 mL), and grinding until a clear solution system was obtained;
2. Slowly adding 40 % SBE-β-CD aqueous solution (5 mL), and the grinding until a clear solution system was obtained;
3. Adding 1 M hydrochloric acid (1 mL), stirring well, then adding ATV014 (150 mg), then grinding and stirring until all ATV014 solid was dissolved;
4. Slowly adding water (13 mL), grinding and stirring until a clear solution was obtained;
5. Slowly dropwise adding 1 M sodium hydroxide solution in batches, measuring a pH with a pH meter and adjusting the pH to 4.

(2) Dose group of ATV014 (100 mg/kg): 3 mL of stock solution of ATV014 at a concentration of 10 mg/mL was aspirated; the mice were infected by nasal drops at day 0, and the drug given at a volume of 0.2 mL/mouse was initiated 2 hours post-challenge, once daily until post-challenge day 3 and day 5.

(3) Dose group of Compound **1** (150 mg/kg): 3 mL of stock solution of Compound **1** at a concentration of 15 mg/mL was aspirated; the mice were infected by nasal drops at day 0, the drug given at a volume of 0.2 mL/mouse was initiated 2 hours post-challenge, once daily until post-challenge day 3 and day 5.

(4) Dose group of ATV014 (100 mg/kg) + Compound **1** (150 mg/kg): 3 mL of stock solution of 10 mg/mL ATV014 and 15 mg/mL Compound **1** was aspirated; the mice were infected by nasal drops at day 0, the drug given at a volume of 0.2 mL/mouse was initiated 2 hours post-challenge, once daily until post-challenge day 3 and day 5.

### 2.4 Monitoring of the weight and survival etc. of mice

The weight of mice was recorded on the day of infection (recorded as Day 0), and then the mice were weighed and the mortality was monitored daily at fixed time points and recorded. General clinical observations were conducted on the mice once a day, including: the motor activity and survival status; eyes and fur; mortality and any observable clinical signs.

Weighing, once a day.

### 2.5 Viral load in the lungs of mice and live virus titer assay

The mice were executed on the third day (recorded as Day 3) and the fifth day (recorded as Day 5) post-infection. The mice were dissected, and the left lungs of the mice were taken, weighed, and put into 1 mL of DPBS buffer for tissue grinding, and the ground tissue fluid was subjected to novel coronavirus titer assay.

### 2.5.1 Determination method of viral load

The operation was as follows: RNA was extracted from the lung tissue, and the viral load was determined by qPCR (quantitative Polymerase Chain Reaction). qPCR was performed using the novel coronavirus (2019-nCoV) Nucleic Acid Detection Kit, Daan Gene (Catalog No.: A0930, primer sequences: DAAN-N-F: AAGAAATTCAACTCCAGGCAGC (SEQ ID NO:1); DAAN-N-R: GCTGGTTCAATCTGTCAAGCAG (SEQ ID NO:2); DAAN-N-P: TCACCGCCATTGCCAGCCA (SEQ ID NO:3)), and the detailed procedure was described in the manual.

### 3. Results

### 3.1 Detection of weight

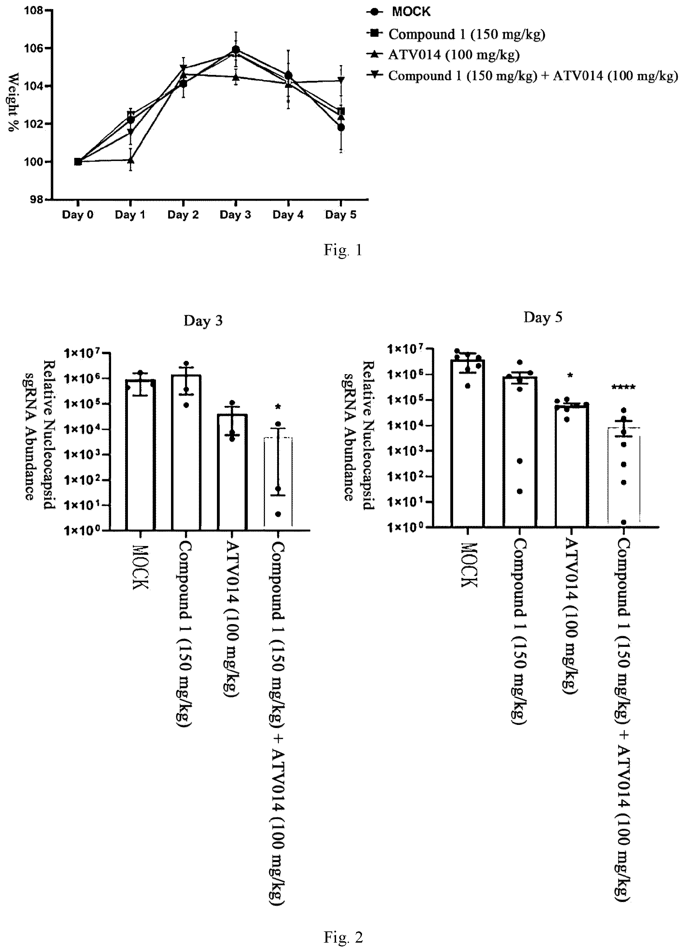

The weight of the infected mice was detected, and the results as shown in Fig. 1 showed that there was no statistical difference in the magnitude of weight change between the different drug-given groups and the vehicle control group.

### 3.2 qPCR detection of viral load in the lungs

On the third and fifth day post-challenge, the mice were executed, and the lungs were removed. The multi-lobed lung tissue was homogenized, RNA of the tissue was extracted, and the results of qPCR detection of viral load in the lungs were shown in Fig. 2.

According to the test results, RNA of novel coronavirus was detected in the lung tissues of all groups of mice. The vehicle control group (Mock) showed high viral loads in all individuals, indicating successful establishment of the infection model in mice. On the day 3 post-infection, only the drug-combination administration group of ATV014 (100 mg/kg) + Compound **1** (150 mg/kg) exhibited a significant antiviral effect, while the viral loads in other drug-treated groups showed no significant difference compared to the control group. On the day 5 post-infection, both the ATV014 (100 mg/kg) group, and the ATV014 (100 mg/kg) + Compound **1** (150 mg/kg) group exhibited significant antiviral effects. Furthermore, the drug-combination administration group of ATV014 (100 mg/kg) + Compound **1** (150 mg/kg) exhibited more significant inhibitory activity in antiviral effects as compared to the monotherapy group.

### 4 Conclusion

Under the present experimental conditions, intragastric administrations of ATV014 and ATV014 + Compound **1** could reduce the viral load to varying degrees in the K18-ACE2 mice, among which ATV014 (100 mg/kg), and ATV014 (100 mg/kg) + Compound **1** (150 mg/kg) achieved significant anti-novel coronavirus effects in the mouse mode. The drug-combination administration group exhibited more significant inhibitory activity as compared to the monotherapy group, indicating that the combination of ATV014 and Compound **1** has a synergistic antiviral effect.

The above examples represent preferred embodiments of the present disclosure. However, the embodiments of the present disclosure are not limited by the above examples. Any variations, modifications, substitutions, combinations, or simplifications made without departing from the spirit and principle of the present disclosure shall be equivalent substitutions, and fall within the scope of protection of the present disclosure.

## Claims

1. A pharmaceutical composition, comprising a first active ingredient and a second active ingredient, wherein the first active ingredient is Compound **1,** an ester of Compound **1,** a salt of Compound **1,** a salt of the ester of Compound **1,** or a combination thereof;
the second active ingredient is ATV014, or a pharmaceutically acceptable salt, a hydrate, or a solvate thereof; and
a molar ratio of the first active ingredient to the second active ingredient ranges from 50:1 to 1:50;

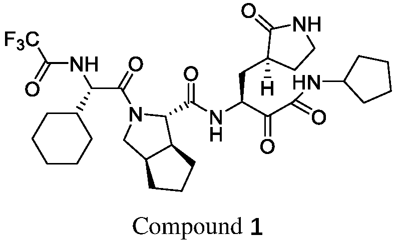

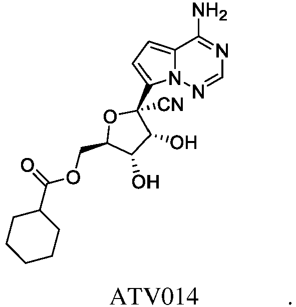

2. The pharmaceutical composition according to claim 1, wherein the first active ingredient is Compound **1,** an ester of Compound **1,** a salt of Compound **1,** a salt of the ester of Compound **1,** or a combination of any two or more in any proportion thereof.

3. The pharmaceutical composition according to claim 1 or 2, wherein the ester of Compound **1** is an ester formed by Compound **1** with an organic acid, and is selected from formate, acetate, propionate, isopropionate, n-butyrate or tert-butyrate of Compound **1;**
the salt of Compound **1** is a salt formed by Compound **1** with an organic acid and/or an inorganic acid, or a salt formed by Compound **1** with an organic base and/or an inorganic base, and is selected from a hydrochloride, a hydrobromide, a sulfate, a phosphate, a benzenesulfonate, a p-toluenesulfonate, a methanesulfonate, a tartrate, a camphorsulfonate, a lithium salt, a sodium salt, a potassium salt, a calcium salt, a magnesium salt, an aluminum salt, an ammonia salt, an ethylenediamine salt, or an triethylamine salt of Compound **1;**
the salt of the ester of Compound **1** is a salt formed by the ester of Compound **1** with an organic base and/or an inorganic base and/or an organic acid and/or an inorganic acid, and is selected from a hydrochloride of methyl ester of Compound **1,** a sulfate of methyl ester of Compound **1,** a hydrochloride of ethyl ester of Compound **1** or a sulfate of ethyl ester of Compound **1.**

4. The pharmaceutical composition according to claim 1, wherein the second active ingredient is selected from ATV014, or a hydrate, or a solvent thereof.

5. The pharmaceutical composition according to claim 1, wherein the molar ratio of the first active ingredient to the second active ingredient ranges from 20:1 to 1:20.

6. The pharmaceutical composition according to any one of claims 1 to 5, wherein the molar ratio of the first active ingredient to the second active ingredient ranges from 10:1 to 1:10, preferably 5:1 to 1:10, and more preferably 1:1.

7. The pharmaceutical composition according to any one of claims 1 to 4, wherein the molar ratio of the first active ingredient to the second active ingredient is 50:1, 40:1, 30:1, 20:1, 10:1, 5:1, 3:1, 2:1, 1.5:1, 1:1, 1:1.5, 1:2, 1:3, 1:5, 1:10, 1:20, 1:30, 1:40, or 1:50.

8. The pharmaceutical composition according to claim 1, wherein in the pharmaceutical composition, the first active ingredient is Compound **1** and/or a sodium salt of Compound **1** and/or a potassium salt of Compound **1** and/or a hydrochloride of Compound **1** and/or a calcium salt of Compound **1** and/or a p-toluenesulfonate of Compound **1;**
the second active ingredient is ATV014, and
the molar ratio of the first active ingredient to the second active ingredient ranges from 50:1 to 1:50.

9. The pharmaceutical composition according to claim 1 or 8, wherein in the pharmaceutical composition, the first active ingredient is Compound **1** and/or a sodium salt of Compound **1** and/or a potassium salt of Compound **1** and/or a hydrochloride of Compound **1** and/or a calcium salt of Compound **1** and/or a p-toluenesulfonate of Compound **1;**
the second active ingredient is ATV014; and
the molar ratio of the first active ingredient to the second active ingredient ranges from 20:1 to 1:20.

10. The pharmaceutical composition according to claim 1 or 8, wherein in the pharmaceutical composition, the first active ingredient is Compound **1** and/or a sodium salt of Compound **1** and/or a potassium salt of Compound **1** and/or a hydrochloride of Compound **1** and/or a calcium salt of Compound **1** and/or a p-toluenesulfonate of Compound **1;**
the second active ingredient is ATV014; and
a molar ratio of the first active ingredient to ATV014 is 50:1, 40:1, 30:1, 20:1, 10:1, 5:1, 3:1, 2:1, 1.5:1, 1:1, 1:1.5, 1:2, 1:3, 1:5, 1:10, 1:20, 1:30, 1:40, or 1:50.

11. The pharmaceutical composition according to any one of claims 1 to 10, wherein the pharmaceutical composition further comprises a third active ingredient, and the third active ingredient is an antiretroviral drug.

12. The pharmaceutical composition according to claim 11, wherein the third active ingredient is ritonavir.

13. The pharmaceutical composition according to claim 11 or 12, wherein a molar ratio of the first active ingredient to the second active ingredient to the third active ingredient ranges from 50:1:0.1-5 to 1:50:0.1-5.

14. The pharmaceutical composition according to any one of claims 11 to 13, wherein the molar ratio of the first active ingredient to the second active ingredient to the third active ingredient ranges from 20:1:0.3-2 to 1:20:0.3-2.

15. The pharmaceutical composition according to any one of claims 11 to 14, wherein the molar ratio of the first active ingredient to the second active ingredient to the third active ingredient ranges from 10:1:0.3-2 to 1:10:0.3-2, preferably 5:1:0.3-2 to 1:10:0.3-2, and more preferably 1:1:0.3-2.

16. The pharmaceutical composition according to any one of claims 11 to 13, wherein the molar ratio of the first active ingredient to the second active ingredient to the third active ingredient is 50:1:1, 40:1:1, 30:1:1, 24:1:1, 20:1:1, 10:1:1, 6:1:1, 5:1:1, 3:1:1, 2:1:1, 1.5:1:1, 1:1:1, 1:1.5:1, 1:2:1, 1:3:1, 1:5:1, 1:6:1, 1:10:1, 1:20:1, 1:24:1, 1:30:1, 1:40:1, or 1:50:1.

17. The pharmaceutical composition according to any one of claims 1 to 16, wherein the first active ingredient and the second active ingredient are present in separate units of formulation, respectively.

18. The pharmaceutical composition according to any one of claims 1 to 17, wherein the first active ingredient and the second active ingredient are administered simultaneously or sequentially.

19. A formulation, comprising the pharmaceutical composition according to any one of claims 1 to 18 and a pharmaceutically acceptable excipient.

20. The formulation according to claim 19, wherein the pharmaceutically acceptable excipient is selected from any one or more of a filler, a binder, a disintegrant, a glidant, a lubricant, a flavoring agent, or in any combination of two or more thereof.

21. The formulation according to claim 20, wherein the filler is any one or more selected from the group consisting of calcium carbonate, magnesium carbonate, calcium phosphate, calcium sulfate, magnesium oxide, calcium carboxymethylcellulose, sodium carboxymethylcellulose, sucrose, lactose, fructose, xylitol, mannitol, starch or derivatives thereof, dextrin, microcrystalline cellulose, or in any combination of two or more in any portion thereof.

22. The formulation according to claim 20, wherein the binder is any one or more selected from the group consisting of gum Arabic, gelatin, tragacanth, dextrin, polyvinylpyrrolidone, starch and derivatives thereof, sodium alginate, sorbitol, syrup, hydroxypropyl methylcellulose, methylcellulose, hydroxypropyl cellulose, hydroxyethyl cellulose, ethyecellulose, sodium carboxymethylcellulose, calcium carboxymethylcellulose, glucose, polymethacrylate, or in any combination of two or more in any portion thereof.

23. The formulation according to claim 20, wherein the disintegrant is any one or more selected from the group consisting of starch, alginate, calcium carboxymethylcellulose, sodium carboxymethylcellulose, croscarmellose sodium, crospolyvinylpyrrolidone, low-substituted hydroxypropyl methylcellulose, microcrystalline cellulose, methylcellulose, or in any combination of two or more in any portion thereof.

24. The formulation according to claim 20, wherein the glidant is any one or more selected from the group consisting of colloidal silicon dioxide, powdered cellulose, magnesium trisilicate, silicon dioxide, talcum powder, or in any combination of two or more in any portion thereof.

25. The formulation according to claim 20, wherein the lubricant is any one or more selected from the group consisting of calcium stearate, glycerol monostearate, glycerol palmitostearate, magnesium stearate, microcrystalline cellulose, sodium benzoate, sodium chloride, sodium dodecyl sulfate, magnesium stearate, sodium stearyl fumarate, talcum powder, zinc stearate, polyethylene glycol, or in any combination of two or more in any portion thereof.

26. The formulation according to claim 20, wherein the flavoring agent is any one or more selected from the group consisting of stevioside, aspartame, other flavors, sweeteners commonly used in the art, or in any combination of two or more in any portion thereof.

27. The formulation according to any one of claims 19-26, wherein the formulation is an oral formulation and is selected from powder, granule, pellet, capsule, tablet, lozenge or oral liquid.

28. Use of the pharmaceutical composition according to any one of claims 1-18 or the formulation according to any one of claims 19-27 in the manufacture of a medicament for relieving or treating novel coronavirus infection.

29. The use according to claim 28, wherein the novel coronavirus infection is caused by a common type of novel coronavirus and its variant strain.

30. The use according to claim 28 or 29, wherein the novel coronavirus infection is caused by an Alpha variant strain, a Beta variant strain, a Gamma variant strain, a Delta variant strain, a Lambda variant strain and/or an Omicron variant strain.

31. The use according to any one of claims 28 to 30, wherein the infection comprises fever, cough, sore throat, pneumonia, acute respiratory infection, severe acute respiratory infection, hypoxic respiratory failure, acute respiratory distress syndrome, sepsis or septic shock.

32. The use according to any one of claims 28 to 31, wherein the first active ingredient and the second active ingredient are separately formulated, and separately administered at a frequency of once a day, twice a day or three times a day.

33. The use according to any one of claims 28 to 31, wherein the first active ingredient and second active ingredient are formulated to be co-delivered in an orally administered dosage unit.

34. The use according to any one of claims 28 to 31, wherein the first active ingredient and the second active ingredient are formulated to be administered in separate orally administered dosage units.
